# EUROPÄISCHE PATENTSCHRIFT

(11) **EP 0 988 002 B1**
(45) Veröffentlichungstag und Bekanntmachung des Hinweises auf die Patenterteilung: **23.07.2003**
(21) Anmeldenummer: 98923972.8
(22) Anmeldetag: 17.06.1998
(51) Int. Cl.: A61F 2/42

(54) **ENDOPROTHESE FÜR EIN GELENK, INSBESONDERE EIN FINGER-, ZEHEN- ODER HANDGELENK**
ENDOPROSTHESIS FOR A JOINT, IN PARTICULAR A FINGER, TOE OR WRIST JOINT
ENDOPROTHESE POUR UNE ARTICULATION, NOTAMMENT UNE ARTICULATION DU DOIGT, DE L'ORTEIL OU DU POIGNET

(30) Priorität: 18.06.1997 CH 148597
(43) Veröffentlichungstag der Anmeldung: 29.03.2000
(73) Patentinhaber: Bähler, André, CH-8032 Zürich (CH); Simmen, Beat R., 8317 Tagelswangen (CH)
(72) Erfinder: Bähler, André, CH-8032 Zürich (CH); Simmen, Beat R., 8317 Tagelswangen (CH)
(74) Vertreter: Hasler, Erich, Dr.
(86) Internationale Anmeldenummer: CH9800262
(87) Internationale Veröffentlichungsnummer: WO98057600

(56) Entgegenhaltungen:
- WO-A-96/25129
- WO-A-96/41596
- DE-A- 3 630 138
- FR-A- 2 736 536
- US-A- 2 765 787
- US-A- 3 899 796
- US-A- 4 349 922
- US-A- 5 534 033

## Beschreibung

### Technisches Gebiet der Erfindung

Die vorliegende Erfindung betrifft eine Endoprothese für ein Gelenk, insbesondere ein Finger-, Zehen- oder Handgelenk gemäss dem Oberbegriff des Patentanspruchs 1.

### Stand der Technik

Das natürliche Metacarpophalangeal-Gelenk (MCP) gibt dem Finger eine seitliche Bewegungsfreiheit, welche je nach Beugung des Gelenkes unterschiedlich ist. In Flexion ist die Bewegungsfreiheit seitlich Null bis lediglich einzelne Grade, in Extension jedoch um 30 Grad. Zudem ist eine begrenzte passive Rotation um die Fingerachse möglich. Das Interphalangeal-Gelenk lässt unabhängig vom Beugewinkel seitlich beinah keinen Bewegungsspielraum frei und die Gelenkteile sind gegeneinander nur sehr wenig rotierbar. So hat jedes Gelenk seine besonderen Freiheitsgrade bezüglich Extension und Flexion, bezüglich Rotation, aber auch bezüglich der Lateroflexion.

Um dem Gelenk die natürlichen Bewegungen von Flexion, Extension und Rotation zu erlauben, während dennoch die Kontrolle über die Bewegungsbahn des Fingers gegenüber der Mittelhand beibehalten ist, wird in der Französischen Patentanmeldung Nr. FR-A1-2 736 536 eine Endoprothese für ein Fingergelenk vorgeschlagen, welche einen hemisphärschen proximalen, einen entsprechenden konkaven distalen Gelenkteil und einen Verbindungs- und "Programmier"-Teil mit einem Stab aus einem geschmeidigen Material aufweist. Der Stab sitzt in beiden Gelenkteilen in einem Führungskanal, welcher im in den Knochenmarkkanal einzusetzenden Stift des Gelenkteils axial angeordnet ist und etwa zylindrisch beginnt, um mit abnehmender Distanz zur Gelenkkopfoberfläche sich trompetenförmig aufzuweiten. Durch das Zusammenwirken von Führungskanal und Verbindungsstab wird der Bewegungsspielraum definiert. Form, Abmessungen und allenfalls die Vorprogrammierung einer Legierung mit "Formgedächtnis" des Verbindungsstabes bestimmen den Bewegungsablauf des Gelenkes mit.

Ein derartiges Gelenk weist den Nachteil auf, dass der geschmeidige Verbindungsstab eine Vielzahl von Aufgaben gleichzeitig erfüllen muss. So muss er in verschiedenen Richtungen unterschiedlich geschmeidig sein, muss abreibfest sein, allenfalls ein Formgedächtnis aufweisen. Er muss eine gewisse Steifheit aufweisen und dennoch flexibel sein. Zudem sollte das Material körperverträglich und langlebig, d.h. nicht ermüdend sein.

In der US-Patentschrift 5,534,033 wird ein orthopädisches Prothese-Implantat vorgeschlagen, bei welchem die Gelenkteile je eine in den Knochenmarkkanal einsetzbare Schale aufweisen, in deren Hohlraum ein Gelenkkopf aus Keramik oder Carbon mittels eines elastomeren Klebers befestigt ist, wobei die Gelenkköpfe aufeinander gleitend sich berühren und die beiden Gelenkteile durch Verbindungsmittel verbunden sind. Die Verbindungsmittel sind entweder eine die Gelenkköpfe umfangende Hülle oder ein Faden, welcher durch ein Loch in den Gelenkköpfen geführt ist. Auch zwei Fäden in Abstand zueinander sind vorgeschlagen. Diese können durch Löcher in den Gelenkköpfen oder auch um den Gelenkkopf herumgeführt sein. Die Funktion der Fäden besteht darin, die Gelenkteile so zu führen, dass sie nicht ausrenken. Es ist dem Dokument nicht zu entnehmen, wie die Verbindungsmittel auf eine Bewegung des Gelenkes reagieren. Es ist zu mutmassen, dass die Verbindungsmittel entweder elastisch sein müssen (gestrickter Dracon) oder an einer Feder befestigt sind. Aus den Zeichnungen und der Beschreibung ist jedenfalls ableitbar, dass sich die Strecke zwischen den Befestigungspunkten des Verbindungsmittels beim Bewegen des Gelenkes in der Länge verändert. Gerade dies aber birgt Nachteile in sich. So können die Fäden, welche durch die Löcher geführt sind, durch die Kanten der Löcher bei der Gelenkkopfoberfläche abgeschert werden, oder sie gelangen wenigstens zwischen die Gelenkköpfe und können dort zerrieben werden. Sind die Fäden um die Gelenkköpfe herumgeführt, reiben sie an den Gelenkköpfen und gewähren bei gebogenem Gelenk keine ausreichende Sicherheit vor dem Ausrenken. Die Hüllen werden bei jeder Beugung auf der Oberseite des Gelenks gestreckt oder es liegt bei gestrecktem Gelenk auf der Oberseite aufgefaltetes Material der Hülle vor.

### Aufgabe der Erfindung

Es ist deshalb Aufgabe der Erfindung eine Endoprothese für kleine Gelenke zu schaffen, bei welcher die erwähnten Nachteile vermieden werden und eine flexible, einen Spielraum gewährende Verbindung beider Gelenkteile gewährleistet ist. Die Verbindung soll so locker sein und so wenig Widerstand leisten, dass die beiden Teile sich so frei bewegen können, wie das natürliche Gelenk. Das Verbindungsstück soll nicht ermüden. Der Bewegungsspielraum des Gelenkes soll, bei gleichzeitig lockerem Zusammenhalt der Gelenkteile, bezüglich Rotation, Extension, Flexion und Lateroflexion, und letztere abhängig von der Extension, definierbar sein. Es soll eine möglichst kleine Abreibung gewährleistet sein.

### Beschreibung der Erfindung

Erfindungsgemäss wird dies durch die Merkmale des Patentanspruchs 1 erreicht. Bei einer erfindungsgemässen Endoprothese besteht eine grosse Bewegungsfreiheit in Beugerichtung und gleichzeitig für eine Verschiebung oder seitliche Auslenkung sowie eine Rotation eine bestimmbare, in der Regel kleinere Freiheit. Dabei ist das Gelenk dennoch geführt und vor Ausrenkung geschützt, denn die Verbindung beider Gelenkteile mit einem Verbindungsstück verhindert eine Luxation.

Beim Beugen von Fasern in Fäden und Geweben oder von flachen Bändern und dünnen Häuten wird das Material aufgrund der kleinen Dimension des Querschnittes in der Richtung der Beugebewegung sehr wenig gestreckt und gestaucht. Fasern, Bänder und Häute sind demzufolge umso geeigneter für flexible, biegbare Verbindungen, je dünner der Materialquerschnitt ist. Diese Eigenschaft von Fasern und Bändern bleibt auch dann erhalten, wenn sie zu grösseren Verbänden versponnen und/oder verwoben werden, um vielfache Zugbelastungen aufnehmen zu können. Solche Verbände können durch Spinnen, Weben oder Knoten gebildet werden und die Form von Schnüren und Seilen, Schläuchen, Bändern oder flächigen Geweben aufweisen. Wesentlich dabei sind die sehr kleinen Abmessungen des Materials in wenigstens einer Dimension für Beugung in einer Richtung, für Beugung in mehreren Richtungen in zwei Dimensionen. Die Belastbarkeit solcher faden-, bandoder hautartiger Teile auf Zug ist hoch auslegbar. Ebenso wird ihre Resistenz gegen Ermüdungsbrüche geschätzt. Hohe Reissfestigkeit mit gleichzeitiger Resistenz gegen Bruch ist z.B. in Schnüren, Seilen, Netzen, Riemen, Schläuchen, Stoffen, Häuten und Folien aller Art verwirklicht und genutzt.

Für Endoprothesen für kleine Gelenke ist die Verwendung solch flexibler faden-, band- oder hautartiger Strukturen als Verbindungsstück zwischen den gegeneinander beweglichen Gelenkteilen deshalb von grossem Vorteil. Ein solches Verbindungsstück ermöglicht eine gewisse Flexibilität des Gelenkes. Verbindende Fäden oder Gewebeteile lassen sich in alle Richtungen beugen und auch verdrehen, ohne dass dadurch die Gefahr einer erhöhten Materialabtragung oder -ermüdung bestehen würde. Ein Spielraum zwischen den Gelenkteilen und dadurch in der Bewegungsfreiheit des Gelenkes kann ohne Einschränkung gewährt werden. Auch führt eine starke Beanspruchung des Gelenkes durch sehr häufiges Beugen und Strecken kaum zu Materialermüdungen.

Bei Gelenken, die zeitlebens durch das Verbindungsstück zusammengehalten werden sollten, besteht das faden- oder gewebeartige Verbindungsstück vorteilhaft aus einem vom Körper nicht resorbierbaren Material.

Zweckmässigerweise besteht zwischen Nut und Verbindungsstück ein seitlicher Spielraum, so dass das Verbindungsstück bei einer seitlichen Bewegung nicht am Gelenkteil abgeschert wird und nicht an der Oberfläche der Nut scheuert.

Zweckmässigerweise können die Gelenkteile durch mehrere faden- und/oder gewebeartigen Verbindungsstücken nebeneinander verbunden sein. Dadurch wird eine seitliche Auslenkung des Gelenkes begrenzt und das Hebelverhältnis zwischen Gelenkteil und Verbindungsstück günstig beeinflusst.

Vorteilhaft liegt eine mit einer Vertiefung, z.B. einer Kehle, Furche oder Nut im konvexen Gelenkteil zusammenwirkende Erhöhung im konkaven Gelenkteil vor und ist durch den gewährten Spielraum zwischen Vertiefung und Erhöhung die seitliche Bewegungsfreiheit des Gelenkes begrenzt. Dadurch liegen an den Berührungspunkten der beiden Gelenkköpfe abriebfeste Materialien vor. Zudem ist die Begrenzung der Bewegungsfreiheit durch die Form der Erhöhung und der Vertiefung ausgestaltbar. Durch eine Vertiefung im einen und eine damit zusammenwirkende Erhöhung im anderen Gelenkkopf kann der Bewegungsspielraum bezüglich Beugung in der Hauptbeugerichtung (Flexion) und Auslenkung quer dazu (Lateroflexion) sehr genau bestimmt werden. Dieser Spielraum ist auch wichtig für die Dauerhaftigkeit der Verankerung der Prothese im Knochen. Er vermindert eine Übertragung von Scher- und Schubkräften auf die Verankerung der Prothese. Diese Kräfte müssen daher in der Regel über die Sehnen und das Kapselgewebe übertragen werden.

Die Oberfläche der Erhöhung bzw. der Vertiefung und die daran angrenzenden Übergänge und die anschliessenden Gleitflächen oder eigentlichen Gelenkflächen zusammen werden als Berührungsfläche bezeichnet. Die Vertiefung kann mit der Nut zusammenfallen, kann aber auch unabhängig davon ausgebildet sein.

Vorteilhaft ist durch den gewährten Spielraum zwischen an die Nut anschliessenden Gleitflächen und mit diesen zusammenwirkenden Gleitflächen des konkaven Gelenkteils die seitliche Bewegungsfreiheit und/oder die Bewegungsfreiheit in Beugerichtung des Gelenkes begrenzt. Je nach Form der Berührungsflächen und je nach Länge und Befestigungspunkt des Verbindungsstückes, bzw. je nach Position des Drehpunkts des Gelenkes bezüglich der Körperachse des konvexen Gelenkteils ist die Bewegungsfreiheit unterschiedlich. So wird die natürliche Kinematik des menschlichen Gelenkes nachgebildet. Für proximale (PIP) und distale (DIP) Interphalangeal-Gelenke (IP-Gelenke) und Zehengelenke sind die Gleitflächen der Gelenkteile vorteilhaft wenigstens teilweise zylindrisch oder kegelstumpfförmig, um eine seitliche Auslenkung der durch das Gelenk verbundenen Glieder zu verhindern. Bevorzugt besteht eine solche Gleitfläche, sowohl für den konvexen wie für den konkaven Gelenkkopf, aus je zwei entgegengesetzt geneigten Kegelstumpfflächen mit gleicher Kegelachse. Dadurch kann zusätzlich eine seitliche Verschiebung der Gelenkteile zueinander verhindert werden.

Allgemeiner ausgedrückt kann gesagt werden, dass zweckmässigerweise beidseitig des Verbindungsstückes wenigstens ein Teil der Gleitflächen bezüglich ihres Querschnitts derart geformt ist, dass die Stellen, bei welchen zur Oberfläche dieser Teile der Gleitflächen senkrechte Linien auf einer Seite des Verbindungsstückes die Körperachse annähern oder schneiden, von den entsprechenden Stellen der entsprechenden Linien auf der anderen Seite des Verbindungsstücks entfernt sind. Und zwar können diese Stellen auf der Seite des Verbindungsstückes liegen, auf der auch die entsprechende Gleitfläche liegt, oder aber auf der anderen Seite. Je nachdem laufen die Gleitflächen gegen die Nut hin so zusammen, dass sie, bei Abwesenheit der Nut, zusammen einen Grat oder aber eine Kehle oder Furche bilden würden. Dadurch wird die Bewegung durch Ausübung von Druck auf die Gleitflächen und Zug auf das Verbindungsstück begrenzt.

Bei einem Winkel zwischen Bewegungsrichtung und Berührungsfläche von 90 Grad ist die Begrenzung der Bewegung eindeutig, bei kleineren Winkeln kann die Grenze durch Strecken des Verbindungsstückes ausgeweitet werden. Die Grenze der Bewegungsfreiheit ist deshalb umso weicher, je kleiner dieser Winkel ist.

Vorteilhaft liegt im konvexen Gelenkteil eine Achse vor, um welche eine Schlaufe des Verbindungsstücks geführt ist. Dadurch wird beim Beugen und Strecken des Gelenks das Verbindungsstück nicht gebeugt, sondern es gleitet, in der Regel ohne Belastung, um die Achse, was bezüglich Abreibung und Ermüdung beinahe keinen Einfluss auf die Alterung des Verbindungsstücks hat. Es kann eine einzige Achse oder in beiden Gelenkteilen je eine Achse vorliegen. Die Achsen können starr oder drehbar im Gelenkteil angeordnet sein.

Vorteilhaft steckt wenigstens eines der Gelenkteile translatorisch beweglich in einer im Knochen befestigten Hülse. Dies verhindert, dass Zugkräfte, welche in der Hand besonders gerne auftreten, auf die Verankerung des Gelenkteils übertragen werden. Zugkräfte müssen daher von den Sehnen und dem Kapselgewebe aufgenommen werden. Dies hat den Vorteil, dass die Endoprothese nicht aus ihrer Verankerung gerissen werden kann. Solche Hülsen können auch so ausgelegt sein, dass eine Rotation der Prothese um die Finger- oder Zehenachse nicht auf das Gelenk übertragen wird, sondern sich dabei das Gelenkteil in der Hülse dreht. Die Montage der Hülsen ist als weiterer Vorteil sehr einfach indem die Hülse in den Markkanal des Knochens eingeschraubt wird.

Zweckmässigerweise ist die Erhöhung je nach zu gewährendem Rotationsspielraum in Beugerichtung länger oder kürzer. Eine längere Erhöhung lässt bei gleichem Spielraum eine kleinere Rotation zu.

Vorteilhaft ist die Nut je nach Beugewinkel breiter oder schmaler, so dass der seitliche Spielraum abhängig von der Gelenkstellung unterschiedlich ausfällt. Dadurch ist die mögliche Lateroflexion in Abhängigkeit der Flexion steuerbar.

Vorteilhaft umgibt die Erhöhung das Verbindungsstück, damit das Verbindungsstück nicht an der Berührungsfläche des Gelenkkopfes, insbesondere der Vertiefung, reiben kann.

Vorteilhaft entspricht in einem Querschnitt quer zur Biegerichtung auf je einer Seite des Gelenkes die durch die Erhöhung und die anschliessende Gleitfläche gebildete Kurve am konkaven Gelenkkopf der durch die Vertiefung und die anschliessende Gleitfläche gebildeten Kurve am konvexen Gelenkkopf. Dadurch liegen die Berührungsflächen bei Belastung wenigstens linear, vorzugsweise jedoch flächig, aneinander an, so dass hohe punktuelle Belastungen und daraus resultierender erhöhter Abrieb vermieden wird.

Vorteilhaft weisen die Körperachse des konvexen Gelenkteils und die Drehachse, um welche das konkave Gelenkteil schwenkbar ist, einen Abstand zueinander auf. Dadurch ist der Spielraum zwischen den Gleitflächen je nach Beugestellung unterschiedlich. Dadurch ist auch die seitliche Bewegungsfreiheit je nach Beugestellung unterschiedlich.

Vorteilhaft die sind Gleitflächen Kugeloberflächen, deren Kugelzentren einen Abstand zueinander aufweisen. Dadurch ist eine flächige Berührung der Gleitflächen praktisch in jedem_Belastungsfall gegeben. Bei einer Flexion ohne Spielraum zwischen den Gleitflächen liegen beide sphärischen Gleitflächen des konkaven Gelenkkopfes flächig an den entsprechenden Gleitflächen des konvexen Gelenkkopfes an und bewegen sich um die Achse durch die beiden Kugelzentren. Bei Lateroflexion und gleichzeitiger Beugung des Gelenkes liegt je nach Richtung der seitlichen Auslenkung die eine oder andere Gleitfläche flächig auf, während die andere Gleitfläche abgehoben ist.

### Kurzbeschrieb der Figuren

Es zeigt:
- Fig. 1: ein einstückig herstellbares Ausführungsbeispiel mit einem bandartigen Verbindungsstück,
a) in der Untersicht und
b) im Vertikalschnitt
- Fig. 2: ein Ausführungsbeispiel mit beidseitig fest verankertem schnur- oder fadenförmigem Verbindungsstück
a) im Längsschnitt und
b) in der Untersicht,
- Fig. 3: ein Ausführungsbeispiel mit einseitig um eine Achse geführtem schnurartigem Verbindungsstück
a) im Längsschnitt und
b) in der Untersicht,
- Fig. 4: eine Endoprothese mit Zapfen und Nut bzw. Erhöhung und Vertiefung, links geschnitten, rechts in Ansicht,
- Fig. 5: eine Endoprothese mit konischen Gleitflächen,
- Fig. 6: eine Endoprothese für das MCP-Gelenk mit sphärischem Gelenkkopf, Zapfen und in gestreckter Stellung vergrössertem seitlichem Spielraum,
a) im Vertikalschnitt,
b) im Horizontalschnitt und
c) den sphärischen Gelenkkopf in der Frontalansicht,
- Fig. 7: eine Fingergelenk-Endoprothese mit zwei reifenförmigen Gleitflächen beidseitig einer Vertiefung,
a) im Vertikalschnitt,
b) im Querschnitt,
- Fig. 8: eine MCP-Gelenk-Endoprothese mit zwei sphärischen Gleitflächen beidseitig der Vertiefung und der Erhöhung,
a) im Vertikalschnitt,
b) im Querschnitt,
- Fig. 9: eine Fingergelenkprothese mit zwei sphärischen Berührungsflächen, wobei jeweils die Gleitfläche und die anschliessende Berührungsfläche der Erhöhung bzw. der Vertiefung zusammen sphärisch sind
a) im Vertikalschnitt,
b) im Querschnitt,
- Fig. 10: eine schematische Darstellung einer Gelenkgeometrie mit einer Berührungsfläche mit vier Kugelzentren.

### Beschrieb der Ausführungsbeispiele

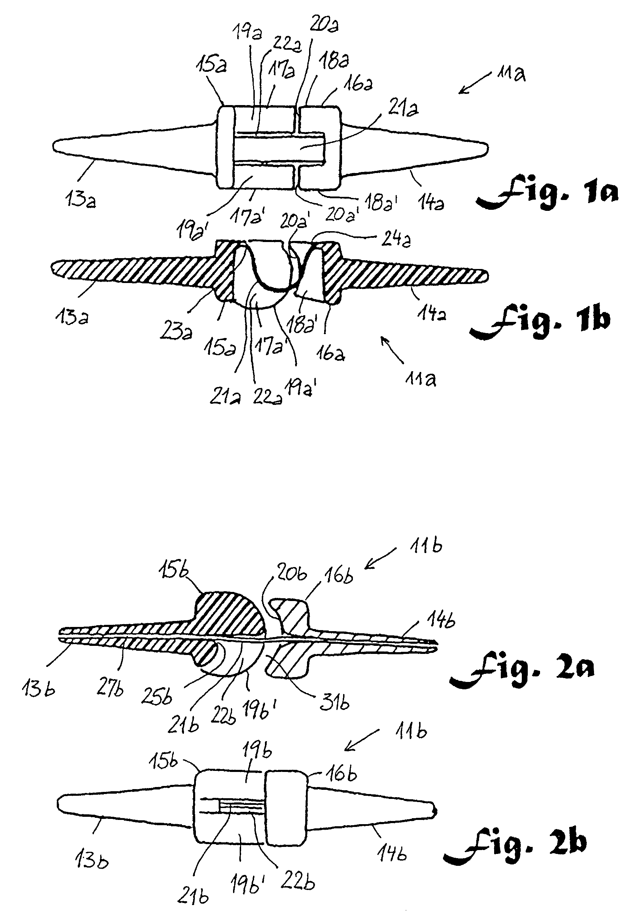

Ein sehr elementares Ausführungsbeispiel einer erfindungsgemässen Endoprothese ist in Figur 1 mit 11a bezeichnet. Es ist einstückig hergestellt und weist zwei Stifte 13a und 14a an zwei Gelenkköpfen 15a und 16a auf. Die Gelenkköpfe 15a,16a weisen seitlich je zwei Kopfstücke 17a,17a' und 18a,18a' auf, welche etwa gegengleich geformte Gleitflächen 19a,19a' respektive 20a,20a' bilden. In einem zentralen Bereich zwischen zwei solchen Paaren von Gleitflächen ist ein bandartiges Verbindungsstück 21a angeordnet. Dieses Verbindungsstück 21a beschreibt eine geschwungene Kurve zwischen den beiden Gelenkköpfen 15a und 16a. Es ist seitlich von den Kopfstücken 17a,17a',18a,18a' getrennt und sein Anfang 23a und sein Ende 24a mit je einem Gelenkkopf 15a,16a verbunden. Der Übergang von bandartigem Verbindungsstück 21a zu Gelenkkopf 15a,16a ist in der Dicke verlaufend. Das bandartige Verbindungsstück 21a wird nun beim Beugen des Gelenkes über dessen ganze Länge verformt. Dank seinem dünnen Querschnitt und der verformbaren Länge des Verbindungsstücks besteht eine geringe Gefahr eines Ermüdungsbruches.

Bei einer seitlichen Auslenkung wird Druck auf zwei Gleitflächen (z.B. 19a und 20a) ausgeübt, während das Verbindungsstück 21a unter Zugbelastung gerät. Dadurch dass das Verbindungsstück 21a in der Nut 22a zwischen den beiden Kopfstücken 17a,17a' geführt ist, ist auch eine seitliche Verschiebung der beiden Gelenkteile 15a und 16a behindert.

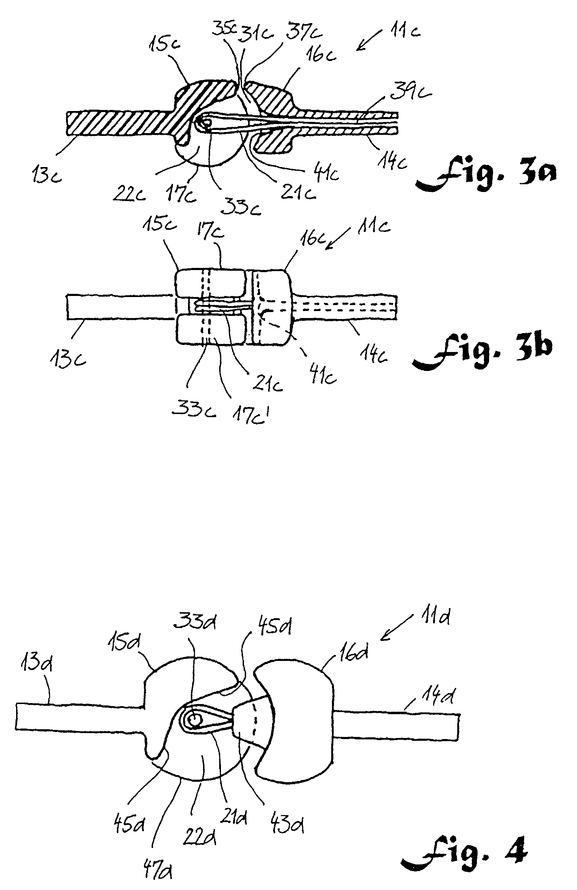

In Figur 2a und b ist ein Ausführungsbeispiel 11b gezeigt, bei dem das Verbindungsstück 21b ein Faden oder eine Schnur ist. Der Faden oder die Schnur ist in beiden Gelenkteilen 15b,16b fest verankert. Bei einer Bewegung des Gelenkes wird das Verbindungsstück 21b gebogen. Durch Wahl des Ortes 25b, an welchem die Schur 21b aus der Befestigungsöffnung 27b im Kopfteil 15b austritt, kann gewählt werden, ob der Spielraum zwischen den Gelenkteilen 15b,16b konstant bleibt oder, je nach Stellung der Gelenkteile 15b,16b zueinander, unterschiedlich ist. Der Faden oder die Schnur 21b läuft im konvexen Gelenkkopf 15b in einem Schlitz 22b. Der Schlitz oder die Nut 22b begrenzt zusammenwirkend mit der Schnur 21b den seitlichen Bewegungsspielraum des Gelenkes 11b. Durch die Flexibilität der Schnur 21b in alle Richtungen und den Spielraum 31b zwischen den Gleitflächen 19b/b' und 20b besteht ein gewisser Bewegungsspielraum auch quer zur Beugerichtung des Gelenkes. Da die Schnur 21b nur Zugkräfte aufnimmt, gleitet in der Regel die Gelenkpfanne bzw. der konkave Gelenkkopf 16b auf dem konvexen Gelenkkopf 15b, ohne dass die Gegenwart eines Verbindungsstückes 21b zwischen den zwei Teilen 15b,16b spürbar ist. Einem Ausrenken des Gelenkes 11b und physiologisch unangebrachten Bewegungen ist mit dem Verbindungsstück 21b jedoch wirksam entgegengewirkt. Bei einer Lateroflexion des Gelenkes stehen die äusseren Bereiche der Gelenkköpfe 15b,16b aneinander an und die Schnur 21b wird gespannt. Bei einer negativen Flexion über die Extensionsstellung hinaus, in Figur 2a also eine Bewegung des Gelenkteils 16b nach oben bzw. im Gegenuhrzeigersinn, werden die Berührungsflächen 19b/19b' und 20b der beiden Gelenkteile gegeneinander geführt. Die Bewegungskurve des Gelenkteils 16b schneidet die Kurve der Berührungsfläche 19b/b' des Gelenkteils 15b, weil der Drehpunkt für die Drehung des Gelenkteils 16b in dieser Richtung nahe der Oberfläche 19b des konvexen Gelenkkopfes 17 liegt.

Die Figur 3 zeigt ein Ausführungsbeispiel 11c einer Endoprothese mit einem konvexen Gelenkkopf 15c und einem konkaven Gelenkkopf 16c. Etwa auf der Körperachse des etwa zylindrischen Gelenkkopfes 15c ist eine Drehachse 33c angeordnet, um welche zwei Fäden oder Schnüre 21c laufen. Diese Schnüre 21c bilden das Verbindungsstück. Das Verbindungsstück 21c läuft in einem Schlitz 22c zwischen den beiden Kopfstücken 17c und 17c'. Anstatt zwei Fäden oder Schnüre 21c könnte auch ein Band, eine einzige oder könnten mehr als zwei Schnüre oder Fäden verwendet werden. Zwischen konvexem Gelenkkopf 15c und konkavem Gelenkkopf 16c ist ein Spielraum 31c vorgesehen, welcher den Gelenkteilen 15c und 16c die Möglichkeit gibt, sich gegeneinander leicht zu verdrehen und seitlich etwas auszulenken bzw. abzuknicken. Auf der Oberseite des Gelenkkopfes 15c ist ein Anschlag 35c angeordnet, welcher zusammenwirkend mit dem oberen Rand 37c des konkaven Gelenkkopfes 16c die Bewegungsfreiheit gegen oben begrenzt. Dadurch dass das Verbindungsstück 21c aus einem flexiblen Material gefertigt ist, kann dem Gelenk ein grosser Spielraum 31c gelassen werden, ohne dass die Gefahr besteht, dass das Verbindungsstück 21c an der Drehachse 33c schabt, es sich verbiegt oder dass das Gelenk ausrenkt. Es sind daher Materialabtragungen und Verformungen der Prothese ebenso verhindert wie auch eine zu präzise Führung und das Ausrenken des Gelenkes. Der Bewegungsspielraum wird durch den Spielraum 31c und die Form der Gelenkköpfe 15c,16c bestimmt.

Das Verbindungsstück 21c ist in der Endoprothese 11c im Stift 14c festgemacht. Die beiden Enden der Schnur 21c wurden in die Öffnung 39c des röhrenförmigen Stiftes 14c eingeführt und darin festgeklemmt, -geschweisst oder -geleimt. Im konkaven Kopfteil 16c ist die Austrittöffnung 41c trichterförmig geöffnet, damit die Schnur 21c nicht an einer scharfen Kante reiben kann. Gegenüber dem in Figur 2 dargestellten Gelenk, weist die in Figur 3 gezeigte Prothese den Vorteil auf, dass das Verbindungsstück 21c beim Beugen des Gelenkes nicht gebogen wird.

Ähnlich ist das in Figur 4 abgebildete Ausführungsbeispiel 11d ausgebildet. Anstatt eines Anschlags (Figur 3a: 35c) begrenzt ein im Schlitz 22d laufender Zapfen 43d zusammenwirkend mit dem Rand 45d des Schlitzes 22d den Bewegungsspielraum der Prothese 11d. Der Zapfen 43d umschliesst die Schnur 21d und hat auch seitlich Spielraum. Es schützt das flexible Verbindungsstück 21d vor Reibung an der Kante 47d des Schlitzes 22d im konvexen Gelenkkopf 15d und begrenzt die seitliche Verschiebung und die Lateroflexion. Der Zapfen 43d ist Teil der konkaven Gelenkpfanne 16d.

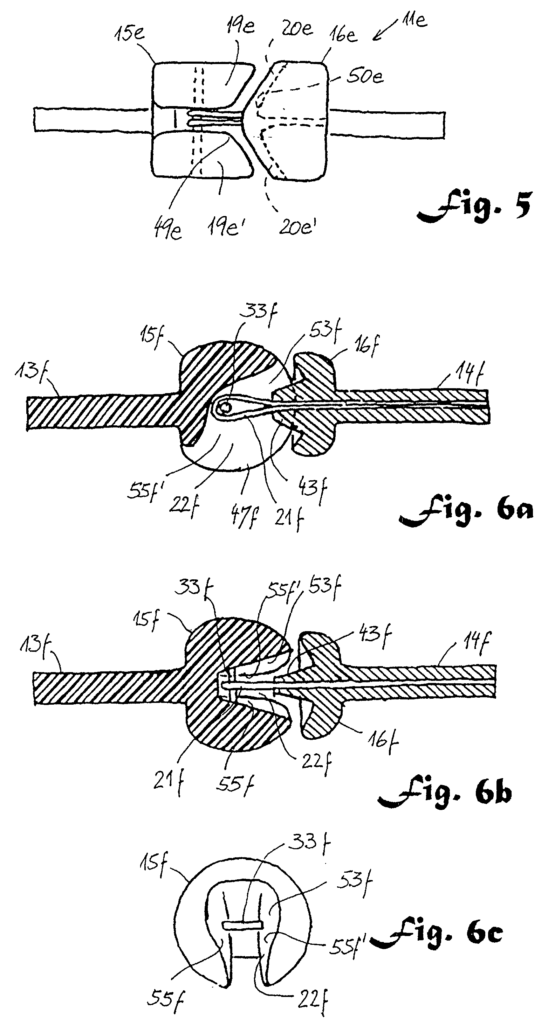

In Figur 5 ist eine weitere Möglichkeit zur Verhinderung der seitlichen Verschiebbarkeit und Begrenzung der Lateroflexion sowie der Rotation eines Gelenkes dargestellt. Die Gleit- oder Berührungsflächen 19e,19e' und 20e,20e' sind Kegelstumpfflächen und sind komplementär geformt. Man könnte auch sagen, der konvexe Gelenkkopf 15e weist eine zentrale Vertiefung 49e und der konkave eine zentrale Erhöhung 50e der Berührungsflächen 19e/e' bzw. 20e/e' auf. Durch die Neigung der Gleit- oder Berührungsflächen bedingt, müssen die Gelenkköpfe 15e und 16e auseinanderweichen, damit eine seitliche Verschiebung der Gelenkköpfe 15e,16e relativ zu einander stattfinden kann. Dieses Auseinanderweichen ist durch das Verbindungsstück 21e verhindert.

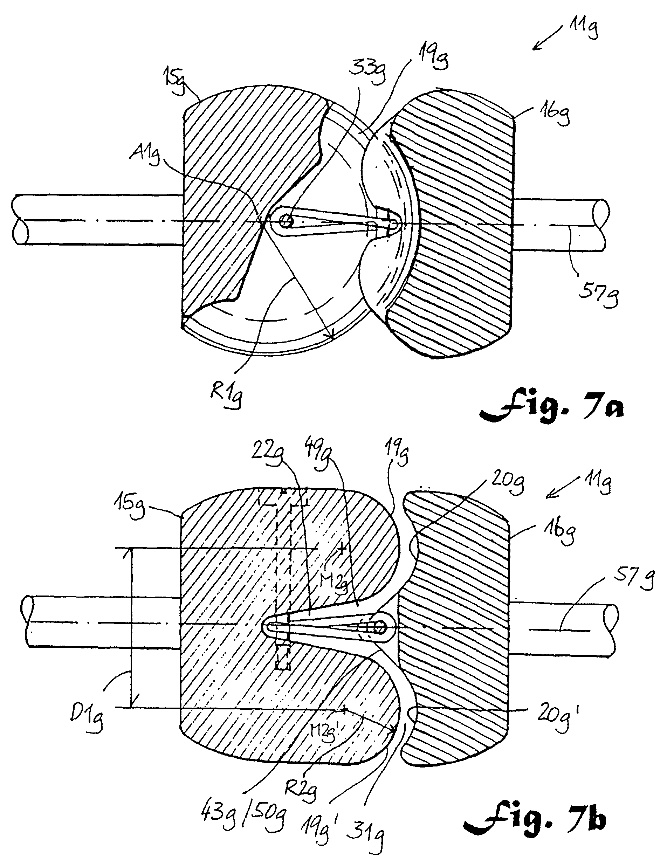

In Figur 6 ist ein den Gelenken in den Figuren 2 bis 4 ähnliches Gelenk 11f dargestellt, welches für das MCP-Gelenk konzipiert ist. Durch seine im Wesentlichen sphärische Form erlaubt der konvexe Gelenkkopf 15f dem mit einer konkaven Pfanne ausgestatteten Gegenstück 16f allseitig ausgelenkt zu werden. Da das natürliche MCP-Gelenk in gestreckter Stellung eine grössere seitliche Auslenkung erlaubt als in gebeugter Stellung, ist die Endoprothese für dieses Gelenk in gleicher Weise ausgelegt. Ein Zapfen 43f begrenzt dazu zusammenwirkend mit dem Rand 47f des Schlitzes 22f den Bewegungsspielraum. Der Schlitz oder die Nut 22f weist nun partiell eine Ausweitung 53f auf (siehe Figur 6c), so dass in einer gestreckten Stellung des Gelenkes der Zapfen 43f mehr Raum für eine seitliche Bewegung erhält. In der gebeugten Stellung hingegen ist zwischen Zapfen 43f und Seitenfläche 55f,55f' weniger Zwischenraum, so dass das Gelenk 11f im Wesentlichen, wie beim natürlichen Gelenk auch, nur in einer Richtung bewegt werden kann.

In Figur 7 ist eine MCP-Gelenkprothese 11g dargestellt, bei welcher die Berührungsflächen 19g,19g', resp. 20g,20g' auf beiden Seiten des Schlitzes 22g oder der Vertiefung 49g bzw. des Zapfens 43g oder der Erhöhung 50g eine Oberflächenform aufweisen, welche in Flexionsrichtung eine Kreisbahn mit einem maximalen Radius R1g um die Achse A1g und quer dazu je eine Kreisbahn mit kleinerem Radius R2g beschreiben. Diese Form gewährleistet, dass trotz des Spielraums 31g zwischen den Gelenkköpfen 15g und 16g praktisch jede Belastung der Berührungsflächen dazu führt, dass diese wenigstens linear aneinander anliegen.

Mit dem Spielraum 31g und den Radien R2g,R2g' sowie den Abständen Dig der Kreiszentren M2g und M2g' zueinander kann nun die seitliche Auslenkbarkeit, die mögliche Rotation und der Spielraum 31g des Gelenks 11g definiert werden. Wenn wie im Beispiel dargestellt, die Flexionsachse A2g des Gelenkes nicht auf der Achse A1g der Berührungsflächen 19g,19g',20g,20g' liegt, ist zudem der Spielraum 31g, und dadurch die maximal mögliche Lateroflexion und Rotation, abhängig von der Beugestellung.

Die Berührungsflächen 20g,20g' des konkaven Gelenkkopfes 16g bilden keine genaue Gegenform der Berührungsflächen 19g,19g'des konvexen Gelenkkopfes 15g, da sonst eine seitliche Auslenkung nicht gut möglich wäre. Die Gleitflächen 20g,20g' sind in der Querschnittlinie durch die Achse 57g des Fingers kreisförmig. Je weiter der Querschnitt von dieser Achse 57g entfernt ist, desto mehr ist die Querschnittlinie nach aussen ausgeweitet, die Kurve abgeflacht. Dadurch liegen unter jedem Auslenkwinkel die Gleitflächen 19g und 20g bzw. 19g' und 20g' jeweils auf einer Kreislinie um die Achse A1g aneinander an.

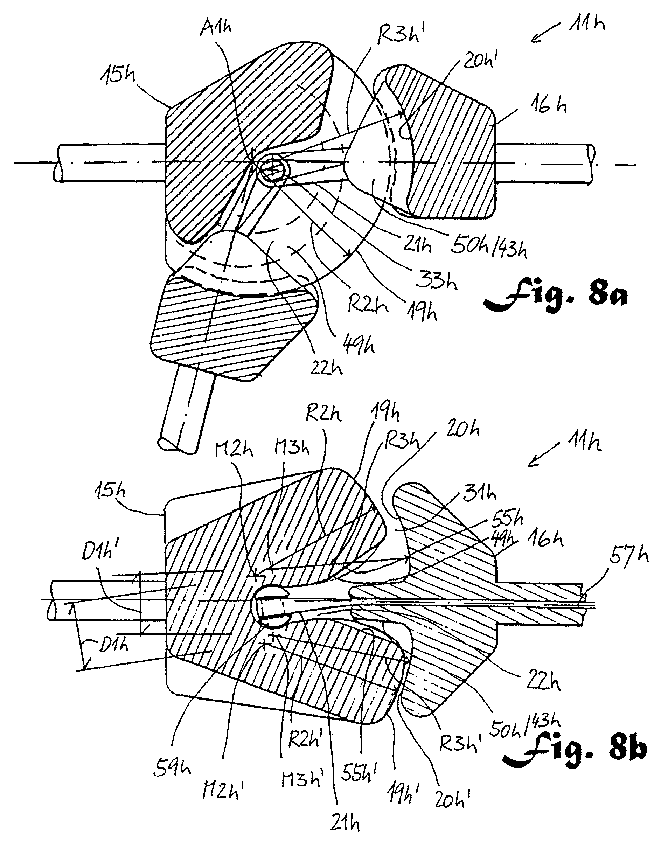

Damit die Gleitflächen bei jedem Lateroflexionswinkel flächig aneinander anliegen, müssen die Gleitflächen sphärisch sein. In der Figur 8 ist eine Gelenkprothese 11h für das MCP-Gelenk des linken Zeigefingers dargestellt, welche je Gelenkteil zwei sphärische Gleitflächen 19h,19h',20h,20h' aufweist. Die Gleitflächen 19h und 19h' haben je ein Kugelzentrum M2h, M2h'. Die Kugelzentren M2h,M2h' weisen einen Abstand D1h zueinander auf. Die gleiche Geometrie haben die beiden konkaven Kugeloberflächen der Gleitflächen 20h,20h'. Ihre Mittelpunkte M3h,M3h' sind mit einem Abstand D1h' zueinander angeordnet, wobei die Längen der Abstände D1h und D1h' sowie der Radien R2h und R3h sowie R2h' und R3h' der Gleitflächen übereinstimmen. Es kann von Vorteil sein, wenn die Radien R2h und R3h gegenüber den Radien R2h' und R3h' kleiner oder grösser sind. In der Praxis werden zudem die Radien der konkaven Gleitflächen 20h,20h' etwas grösser sein als die Radien der konvexen Gleitflächen 19h,19h', da sich beim implantierten Gelenk 11h zwischen den Gleitflächen 19h/19h' einerseits und 20h/20h' andererseits ein Flüssigkeitsfilm einstellen soll.

Die Achse 33h für die Verbindungsschlaufe 21h liegt etwas neben der Achse A1h durch die Mittelpunkte M2h und M2h', so dass der Spielraum 31h zwischen den Gleitflächen je nach Beugewinkel kleiner oder grösser ist. Der grösste Spielraum 31h ist im Beispiel bei einer Beugestellung von ca. 15 Grad. Der Spielraum 31h wird bei etwa 100 Grad zu Null, so dass das Gelenk nicht weiter gebeugt werden kann.

Entsprechend dem vorhandenen Spielraum 31h ist die seitliche Auslenkbarkeit kleiner oder grösser. Wenn sich die Gleitflächen bei 100 Grad Beugung berühren, ist die mögliche Lateroflexion gegen Null, bei gestrecktem oder bis zu 15 Grad leicht gebogenem Finger etwa 45 Grad.

Die Achse A1h durch die Mittelpunkte M2h und M2h' verläuft in einem Winkel von ca. 97,5 Grad quer zur gestreckten Fingerachse 57h. Dadurch ist der Finger von der Gestreckten auf die Seite der Gleitflächen 19,20 um 15 Grad mehr auslenkbar als auf die Seite der Gleitflächen 19',20'. Bei einer Belastung des Gelenks, bei welcher der Finger gegen die Mittelhand gedrückt wird, wird also zuerst Druck auf die Gleitflächen 19',20' ausgeübt. Dadurch wird der Finger zur Handmitte hin abgelenkt. Auch eine Flexion lässt den Finger zur Handmitte hin tendieren. Die natürlichen Gelenkachsen werden durch die Gelenkkonstruktion optimal respektiert.

Die Achse 33h ist durch ein in einem sphärischen Hohlraum einklinkbaren und darin drehbaren Körper 59h mit sphärischer Kontaktfläche zum Hohlraum gebildet. Dadurch ist die Achse 33h in alle Richtungen verdrehbar. Die Schlaufe 21h ist flach um die Achse 33h geführt und im konkaven Gelenkteil 16h, insbesondere in seinem Stamm oder Stiel 14h verankert. Die Schlaufe nimmt Veränderungen der Position der Achse 33h bezüglich der Fingerachse 57h auf, indem sie sich verformt. Bei gestrecktem Finger ist die Schlaufe 21h lose, lediglich bei starker Lateroflexion oder Flexion verhindert sie eine weitergehende Bewegung der Gelenkteile 15h,16h zueinander und daher auch eine Luxation.

Die Erhöhung 50h ist entsprechend der Flanken 55h/55h' der Vertiefung 49h ausgebildet, so dass bei einer Auslenkung des konkaven Gelenkteils 16h bis zum Anschlagen der Erhöhung 50h an der Flanke 55h diese sich flächig berühren.

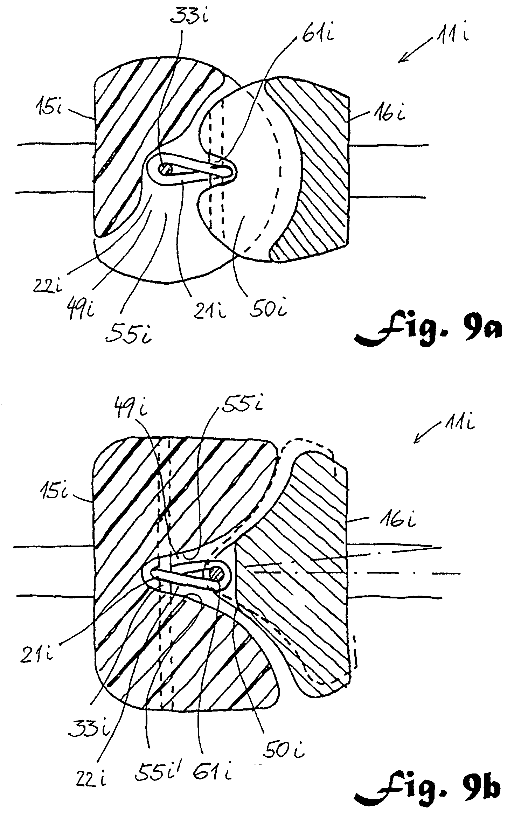

Für Gelenke mit kleinerer Lateroflexion, wie proximale oder distale Interphalangeal-Gelenke (PIP,DIP) wird eine flächige Berührung, ein grosser Spielraum zwischen den Gelenkteilen und eine kleine Lateroflexion erreicht, wenn die Kugelmittelpunkte, wie in der Figur 9 dargestellt, möglichst auseinandergerückt sind. Wenn der Abstand der Kugelmittelpunkte grösser als die Summe der beiden Kugelradien ist, entsteht eine Vertiefung, deren Flanken 55i/55i' auf der Kugeloberfläche liegen. In Figur 9 sind zudem zwei Achsen 33i und 61i für die Verbindungsschlaufe 21i dargestellt. Die Schlaufe 21i ist ringförmig. Sie ist um zwei senkrecht zueinander angeordnete Achsen 33i im konvexen Gelenkteil 15i und 61i im konkaven Gelenkteil 16i geführt.

Weiter sind auch Gelenke im Rahmen der Erfindung ausgestaltbar, welche drei oder gar vier und mehr vorzugsweise sphärische Gleitflächen aufweisen. Dadurch lässt sich die Berührungsfläche zwischen den beiden Gelenkteilen während einer Lateroflexion und bei seitlich ausgelenkter Stellung gross halten. Dies ist beispielsweise für das Handgelenk von Interesse.

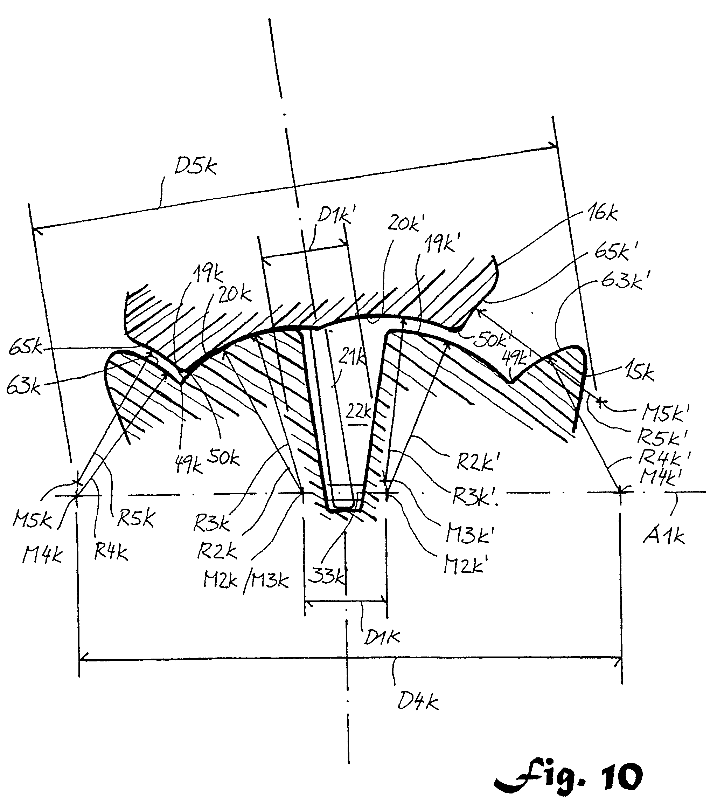

Eine solche Handgelenkprothese 11k ist in Fig. 10 im Querschnitt schematisch dargestellt. Wiederum sind die Radien R2k, R2k', R4k, R4k' der Gleitflächen 19k,19k',63k,63k' des konvexen Gelenkteils 15k entsprechend den Radien R3k,R3k',R5k,R5k' der Gleitflächen 20k,20k',65k,65k' des konkaven Gelenkteils 16k. Die Abstände D1k und D1k' zwischen den Mittelpunkten M2k und M2k' bzw. M3k und M3k' sind gleich gross. Der Abstand D4k zwischen M4k und M4k' ist jedoch grösser als der Abstand D5k zwischen M5k und M5k'. Bei einer Lateroflexion bewegt sich das Gelenkteil 16k jeweils um den Mittelpunkt M3k oder M3k' bis die Gleitflächen 63k und 65k bzw. 63k' und 65k' aneinander anstehen. Die Kugelmittelpunkte aller Gleitflächen liegen beim konvexen Gelenkteil 15k auf einer Achse A1k, beim konkaven auf zwei sich im Zentrum des Gelenks schneidenden Achsen. Dadurch geschieht die Flexion des Gelenkes immer um die gleiche Achse A1k durch die Mittelpunkte M4k, M2k, M2k', M4k'. Dennoch ist das konkave Gelenkteil 16k seitlich verschwenkbar.

Je näher die auf einer Seite liegenden Mittelpunkte, z.B. M2k und M4k, einander sind, desto weicher ist die Grenze der seitlichen Bewegungsfreiheit. Bei geeignetem Spielraum ist gar ein Gleiten über mehrere Kugelflächen nebeneinander möglich, wobei lediglich höchstens zwei benachbarte Kugelflächen in Berührung mit den entsprechenden Flächen des konkaven Gelenkteils stehen.

Es ist denkbar, dass z.B. bei einem Handgelenk das eine Gelenkteil aus zwei Teilen besteht, welche allenfalls mittels schnurartigem Verbindungsstück verbunden sind. Die Teile sind dadurch gegeneinander leicht verschwenkbar. Das eine könnte dann an der Elle, das andere an der Speiche festgemacht sein.

Zusammenfassend kann gesagt werden, dass bei einer Endoprothese (11) für ein Gelenk die beiden zusammenwirkenden Gelenkteile (15,16) durch ein schnurartiges Verbindungsstück (21) verbunden sind , welches in der Nähe der Körperachse (A1, z.B. M2h,M2h') des konvexen Gelenkkopfes (15) befestigt ist und sich durch eine in Beugerichtung des Gelenkes längliche Nut (22) erstreckt. Das Verbindungsstück gewährt einen Spielraum (31) zwischen den Berührungsflächen (19,20) des Gelenks (11). Es ist durch eine Erhöhung (50,43) im konkaven Gelenkteil (16) vor Friktion an der Nutwand (55) geschützt. Eine Erhöhung (43,50) am konkaven Gelenkteil (16) und eine Vertiefung (49) am konvexen Gelenkteil (15) wirken so zusammen, dass der seitliche Bewegungsspielraum zwischen Vertiefung und Erhöhung die Bewegungsfreiheit bezüglich Lateroflexion des Gelenkes bestimmt. In vorteilhaften Ausführungsformen liegt dank sphärischer Oberflächen bei Belastung in jeder Stellung des Gelenkes wenigstens ein Paar von entsprechenden Gleitflächen (z.B. 19,20...) auf den beiden Gelenkköpfen flächig aufeinander auf.

### Bezugsziffernachweis:

- a,b...: Ausführungsbeispiele
- a,a': entsprechende Teile auf der anderen Seite des Verbindungsstückes am gleichen Gelenkkopf
- 11: Endoprothese
- 13: Stiel am konvexen Gelenkkopf zur Implantierung in den Knochenmarkkanal
- 14: Stiel am konkaven Gelenkkopf zur Implantierung in den Knochenmarkkanal
- 15: Konvexer Gelenkkopf
- 16: Konkaver Gelenkkopf
- 17: Kopfteil auf einer Seite
- 18: Kopfteil auf der anderen Seite
- 19: Gleitflächen am konvexen Gelenkkopf
- 20: Gleitflächen am konkaven Gelenkkopf
- 21: Verbindungsstück, Schnur, Band...
- 22: Schlitz, Nut
- 23: Anfang des Verbindungsstückes
- 24: Ende des Verbindungsstückes
- 25: Austrittstelle des Verbindungsstückes aus dem Befestigungskanal
- 27: Befestigungskanal
- 31: Spielraum zwischen den Gleitflächen
- 33: Drehachse für das Verbindungsstück
- 35: Anschlag
- 37: Rand am konkaven Gelenkteil
- 39: Befestigungsöffnung
- 41: ausgerundete Austrittöffnung
- 43: Zapfen, Erhöhung
- 45: Rand des Schlitzes, der Nut, Anschlag für Zapfen/Erhöhung
- 47: Kante, Rand des Schlitzes, der Nut, der Vertiefung
- 49: Vertiefung, Furche, Kehle
- 50: Erhöhung, Grat
- 53: Ausweitung, Verbreiterung der Nut
- 55: Seitenwand, Nutwand, Seitenfläche, Flanke der Nut
- 57: Achse des Fingers
- 59: einklinkbarer Körper mit Achse für Verbindungsstück
- 61: Achse für Verbindungsstück am konkaven Gelenkkopf
- 63: weitere Gleitfläche am konvexen Gelenkkopf
- 65: weitere Geleitfläche am konkaven Gelenkkopf

- A1: Körperachse des konvexen Gelenkkopfes
- M2: Mittelpunkt für Bogen der konvexen Gleitflächen
- M3: Mittelpunkt für Bogen der konkaven Gleitflächen
- M4: Mittelpunkt für weitere konvexe Geleitfläche
- M5: Mittelpunkt für weitere konkave Geleitfläche
- R2: Radius einer Gleitfläche von M2 ausgehend
- R3: Radius einer Gleitfläche von M3 ausgehend
- R4: Radius einer Gleitfläche von M4 ausgehend
- R5: Radius einer Gleitfläche von M5 ausgehend

## Patentansprüche

1. Endoprothese für ein Gelenk (11a-k), insbesondere ein Finger-, Zehen- oder Handgelenk, mit einem proximalen (15a-k) und einem distalen Gelenkteil (16a-k), von welchen ein Gelenkteil (15a-k) in Beugerichtung eine im Wesentlichen um eine Körperachse konvexe Berührungsfläche (19a-k,19a-k') aufweist, in diesem konvexen Gelenkteil (15a-k) eine in Beugerichtung langgestreckte Nut (22a-k,49g,h,i) ausgebildet ist, und das andere Gelenkteil (16a-k) eine entsprechend im Wesentlichen konkave Berührungsfläche (20a-k,20a-k') aufweist,
wobei die Gelenkteile (15a-k,16a-k) mit einem flexiblen, Zugkräfte übertragenden Verbindungsstück (21a-k) verbunden sind, welches zwischen den Berührungsflächen (19a-k/19ak',20a-k/20a-k',55h,h'i,i',63k,k',65k,k') einen bestimmten Spielraum (31a-k) gewährt,
das Verbindungsstück (21a-k) in der im konvexen Gelenkteil (15a-k) ausgebildeten Nut oder am Grund dieser Nut (22a-k,49g,h,i) befestigt ist, und
zwischen Nut (22a-k,49g,h,i) und Verbindungsstück (21a-k) ein seitlicher Spielraum besteht,
**dadurch gekennzeichnet,**
**dass** das Verbindungsstück (21a-k) ein faden- oder bandartiges Element (21a-k) mit einer bestimmten Länge aufweist,
und **dass** wenigstens die seitliche Bewegungsfreiheit des Gelenkes (11d-k) begrenzt ist,
- durch den gewährten Spielraum zwischen einer Vertiefung (22d-k,49e,g,k), z.B. einer Kehle (49e,g,h,i,k,k'), Furche oder Nut (22d,f,g), welche im konvexen Gelenkteil (15d-k) vorliegt, und einer mit dieser zusammenwirkenden Erhöhung (43d,f,g,50g,e,h,i,k,k'), welche am konkaven Gelenkteil (16d-k) vorliegt, und/oder
- durch den durch die Länge des faden- oder bandartigen Elements (21a-k) gewährten Spielraum (31a-k) zwischen an die Nut (22a-k) anschliessenden Gleitflächen (19a-k,a'-k',63k,k') des konvexen Gelenkteils (15a-k) und mit diesen zusammenwirkenden Gleitflächen (20a-k,a'-k', 65k,k') des konkaven Gelenkteils (16a-k) und die Form der Gleitflächen.

2. Endoprothese nach einem der Ansprüche 1, **dadurch gekennzeichnet, dass** in der Nut (22c-k) eine Achse (33c-k) angeordnet ist, um welche eine Schlaufe des Verbindungsstücks (21c-k) geführt ist.

3. Endoprothese nach einem der Ansprüche 1 bis 2, **dadurch gekennzeichnet, dass** die Nut (22f,h) je nach Beugewinkel breiter oder schmaler ist, so dass die seitliche Bewegungsfreiheit abhängig von der Gelenkstellung unterschiedlich ausfällt.

4. Endoprothese nach einem der Ansprüche 1 bis 3, **dadurch gekennzeichnet, dass** die Erhöhung (43d,f,h) das fadenoder bandartige Element (21d,f,h) umgibt.

5. Endoprothese nach einem der Ansprüche 1 bis 4, **dadurch gekennzeichnet, dass** in einem Querschnitt quer zur Biegerichtung auf je einer Seite des Gelenkes (11e-k) die durch die Erhöhung (43f,g,h,50e,i,k,k') und die anschliessende Berührungsfläche (20e-k,e'-k', 65k,k') gebildete Kurve am konkaven Gelenkkopf (16e-k) der durch die Vertiefung (22f,g,h, 49e,i,k,k') und die anschliessende Berührungsfläche (19e-k,e'-k', 63k,k') gebildeten Kurve am konvexen Gelenkkopf (15e-k) entspricht.

6. Endoprothese nach einem der Ansprüche 1 bis 5, **dadurch gekennzeichnet, dass** die Erhöhung (43d, f-h, 50i,k,k') je nach zu gewährendem Rotationsspielraum in Beugerichtung länger oder kürzer ist.

7. Endoprothese nach einem der Ansprüche 1 bis 6, **dadurch gekennzeichnet, dass** die Körperachse (z.B. Alg,h,k) des konvexen Gelenkteils (15b-k) und die Drehachse (33b,b'ck), um welche das konkave Gelenkteil (16b-k) schwenkbar ist, einen Abstand zueinander aufweisen.

8. Endoprothese nach einem der Ansprüche 1 bis 7, **dadurch gekennzeichnet, dass** beidseitig des Verbindungsstückes (21a-c,e,g-k) wenigstens ein Teil der Gleitflächen (19a-c,e,g-k, 20a-c,e,g-k, 63k,k', 65k,k') bezüglich ihres Querschnitts derart geformt ist, dass die Stellen (z.B. M2h,M2k,M5k,), bei welchen zur Oberfläche dieser Teile der Gleitflächen senkrechte Linien auf einer Seite des Verbindungsstückes die Körperachse annähern oder schneiden, von den entsprechenden Stellen (z.B. M2h',M2k',M5k') der entsprechenden Linien auf der anderen Seite des Verbindungsstücks entfernt sind.

9. Endoprothese nach einem der Ansprüche 1 bis 8, **dadurch gekennzeichnet, dass** die Gleitflächen Kugeloberflächen (19h-k,h'-k',20h-k,h'-k',63k,k',65k,k') aufweisen, deren Kugelmittelpunkte (M2h,M2h',M2k,M2k',M5k,M5k') einen Abstand zueinander aufweisen.

## Claims

1. Endoprosthesis for a joint (11a-k), particularly a finger, toe or hand joint, with a proximal (15a-k) and a distal joint part (16a-k), of which one joint part (15a-k) in the flexion direction has an essentially convex contact surface (19a-k, 19a-k') around a body axis, in said convex joint part (15a-k) being formed in the flexion direction a longitudinally extended groove (22a-k, 49g, h, i), and the other joint part (16a-k) has a corresponding essentially concave contact surface (20a-k, 20a-k'), whereby the joint parts (15a-k, 16a-k) are joined with a flexible connection piece (21a-k) taking up tensile forces, which connection piece assures a defined play space (31a-k) between the contact surfaces (19a-k/19a-k', 20a-k/20a-k', 55h, h' i, i', 63k, k', 65k, k'), the connection piece (21a-k) being fastened in this groove or at the base of this groove (22a-k, 49g, h, i), and having a lateral play space between groove (22a-k, 49g, h, i) and connection piece (21a-k)
**characterized in that**
the connection piece (21a-k) has a thread-type or tape-type element (21a-k) having a defined length,
and **in that** at least the lateral freedom of movement of joint (11d-k) is limited
- due to the assured play space between a depression (22d-k, 49e, g-k), e.g., a channel (49e, g, h, i, k, k'), a furrow or a groove (22d, f, g) in convex joint part (15d-k) and an elevation (43d, f, g, 50g, e, h, i, k, k') in the concave joint part (16d-k) cooperating with the depression, and/ or
- due to the play space (31a-k) assured by the length of the thread-type or tape-type element (21a-k) between sliding surfaces (19a-k, a'-k', 63k, k') of the convex joint part (15a-k) adjacent to the groove (22a-k) and sliding surfaces (20a-k, a'-k', 65k, k') of the concave joint part (16a-k) cooperating with them and to the form of the sliding surfaces.

2. Endoprosthesis according to one of claims 1, further **characterized in that** an axis (33c-k) is arranged in groove (22c-k), and a loop of connection piece (21c-k) is guided around this axis.

3. Endoprosthesis according to one of claims 1 to 2, further **characterized in that** groove (22f, h) is wider or narrower depending on the angle of flexion, so that the lateral freedom of movement is different depending on the joint position.

4. Endoprosthesis according to one of claims 1 to 3, further **characterized in that** elevation (43d, f, h) surrounds the thread-type or tape-type element (21d, f, h).

5. Endoprosthesis according to one of claims 1 to 4, further **characterized in that** the curve at the concave condyle (16e-k) formed by elevation (43f, g, h, 50e, i, k, k') and the adjacent contact surface (20e-k, e'-k', 65k, k') corresponds to the curve at the convex condyle (15e-k) formed by the depression (22f, g, h, 49e, i, k, k') and the adjacent contact surface (19e-k, e'-k', 63k, k') in a cross section crosswise to the bending direction on one side of joint (11e-k).

6. Endoprosthesis according to one of claims 1 to 5, further **characterized in that** elevation (43d, f-h, 50i, k, k') is longer or shorter, each time depending on the rotation play space to be guaranteed in the flexion direction.

7. Endoprosthesis according to one of claims 1 to 6, further **characterized in that** the body axis (e.g. A1g, h, k) of convex joint part (15b-k) and the axis of rotation (33b, b' c-k), around which concave joint part (16b-k) can be pivoted, are distanced from one another.

8. Endoprosthesis according to one of claims 1 to 7, further **characterized in that** at least one part of sliding surfaces (19a-c, e, g-k, 20a-c, e, g-k, 63k, k', 65k, k') on both sides of connection piece (21a-c, e, g-k) is formed with respect to its cross section such that places (e.g., M2h, M2k, M5k), at which lines perpendicular to the surface of these parts of the sliding surfaces approach or intersect the body axis on one side of the connection piece, are distanced from the corresponding places (e.g., M2h', M2k', M5k') of the corresponding lines on the other side of the connection piece.

9. Endoprosthesis according to one of claims 1 to 8, further **characterized in that** the sliding surfaces have spherical surfaces (19h-k, h'-k', 20h-k, h'-k", 63k, k', 65k, k') whose spherical central points (M2h, M2h', M2k, M2k', M5k, M5k') are at a distance relative to one another.

## Revendications

1. Endoprothèse pour une articulation (11a-k), en particulier une articulation de doigt, de doigt de pied, ou une articulation de la main, avec une partie jointive proximale (15a-k) et une partie jointive distale (16a-k), dont une partie jointive (15a-k) dans la direction de flexion présente une surface de contact essentiellement convexe (19a-k, 19a-k') autour d'un axe de corps, dans ladite partie jointive convexe (15a-k) formée dans le sens de la flexion une rainure étendue longitudinalement (22a-k, 49g, h, i), et l'autre partie jointive (16a-k) présente une surface de contact correspondante essentiellement concave (20a-k, 20a-k'), tandis que les parties jointives (15a-k, 16a-k) sont reliées par une pièce de connexion flexible (21a-k) encaissant les forces de traction, laquelle pièce de connexion assure un jeu défini (31a-k) entre les surfaces de contact (19a-k/19a-k', 20a-k/20a-k', 55h, h', i, i', 63k, k', 65k, k'), la pièce de connexion (21a-k) étant fixée dans cette rainure ou à la base de cette rainure (22a-k, 49g, h, i) et ayant un jeu latéral entre la rainure (22a-k, 49g, h, i) et la pièce de connexion (21a-k),
**caractérisé en ce que**
la pièce de connexion (21a-k) comporte un élément de type filetage ou ruban (21a-k) ayant une longueur définie,
et **en ce que** la liberté latérale du mouvement d'articulation (11d-k) est limitée
- par le jeu assuré entre une dépression (22d-k, 49e, g-k), par exemple un canal (49e, g, h, i, k, k'), un sillon ou une rainure (22d, f, g) dans la partie jointive convexe (15d-k) et une partie relevée (43d, f, g, 50g, e, h, i, k, k') dans la partie jointive concave (16d-k) coopérant avec la dépression et/ou
- par le jeu (31a-k) assuré par longueur de l'élément de type filetage ou ruban (21a-k) entre les surfaces coulissantes (19a-k, a'-k', 63k, k') de la partie jointive convexe (15a-k) adjacente à la rainure (22a-k) et les surfaces coulissantes (20a-k, a'-k', 65k, k') de la partie jointive concave (16a-k) coopérant avec elles et la forme des surfaces coulissantes.

2. Endoprothèse selon la revendication 1, **caractérisée en outre par le fait qu'**un axe (33c-k) est disposé dans la rainure (22c-k) et qu'une boucle de la pièce de connexion (21c-k) est guidée autour de cet axe.

3. Endoprothèse selon l'une des revendications 1 ou 2, **caractérisée en ce que** la rainure (22f, h) est plus large ou plus étroite selon l'angle de flexion, de sorte que la liberté latérale de mouvement est différente selon la position de l'articulation.

4. Endoprothèse selon l'une des revendications 1 à 3, **caractérisée en outre en ce que** la partie relevée (43d, f, h) entoure l'élément de type filetage ou ruban (21d, f, h).

5. Endoprothèse selon l'une des revendications 1 à 4, **caractérisée en outre en ce que** la courbe au niveau du condyle concave (16e-k) formé par la partie relevée (43f, g, h, 50e, i, k, k') et de la surface de contact adjacent (20e-k, e'-k', 65k, k') correspond à la courbe au niveau du condyle convexe (15e-k) formé par la dépression (22f, g, h, 49e, i, k, k') et la surface de contact adjacente (19e-k, e'-k', 63k, k') dans une section transversale par rapport à la direction de torsion sur un côté de l'articulation (11e-k).

6. Endoprothèse selon l'une des revendications 1 à 5, **caractérisée en outre en ce que** la partie relevée (43d, f-h, 50i, k, k') est plus longue ou plus courte, dépendant à chaque fois du jeu de rotation à garantir dans le sens de la flexion.

7. Endoprothèse selon l'une des revendications 1 à 6, **caractérisée en outre en ce que** l'axe du corps (par exemple A1g, h, k) de la partie jointive convexe (15b-k) et l'axe de rotation (33b, b', c-k) autour duquel la partie jointive concave (16b-k) peut pivoter, sont écartées l'une de l'autre.

8. Endoprothèse selon l'une des revendications 1 à 7, **caractérisée en outre en ce qu'**au moins une partie des surfaces coulissantes (19a-c, e, g-k, 20a-c, e, g-k, 63k, k', 65k, k') des deux côtés de la pièce de connexion (21a-c, e, g-k) est formée par rapport à sa section transversale de sorts que les points (par exemple M2h, M2k, M5k), au niveau desquels des lignes perpendiculaires à la surface de ces pièces des surfaces coulissantes s'approchent de l'axe de corps ou coupent ce dernier d'un côté de la pièce de connexion, sont écartées des points correspondants (par exemple M2h', M2k', M5k') des lignes correspondantes de l'autre côté de la pièce de connexion.

9. Endoprothèse selon l'une des revendications 1 à 8, **caractérisée en ce que** les surfaces coulissantes présentent des surfaces sphériques (19h-k, h'-k', 20h-k, h'-k", 63k, k', 65k, k') dont les centres (M2h, M2h', M2k, M2k', M5k, M5k') sont écartés les uns des autres.
